# EUROPEAN PATENT APPLICATION

(11) **EP 2 509 130 A1**
(43) Date of publication of application: **10.10.2012**
(21) Application number: 10834533.1
(22) Date of filing: 29.11.2010
(51) Int. Cl.: H01L 51/50, C09K 11/06, H05B 33/10

(54) **ORGANIC EL ELEMENT AND METHOD FOR MANUFACTURING ORGANIC EL ELEMENT**

(30) Priority: 03.12.2009 JP 2009275265
(71) Applicant: Toray Industries, Inc., Tokyo 103-8666 (JP)
(72) Inventor: JO, Yukari, Otsu-shi Shiga 520-8558 (JP); SHIRASAWA, Nobuhiko, Otsu-shi Shiga 520-8558 (JP); FUJIMORI, Shigeo, Otsu-shi Shiga 520-8558 (JP)
(74) Representative: Hoefer & Partner
(86) International application number: PCT/JP2010/071222
(87) International publication number: WO 2011/068083

(57) **Abstract**

Disclosed is an organic El element containing a specific naphthacene derivative, and by setting the amount of a precursor compound of the naphthacene derivative contained in the organic EL element to a certain level or less, the organic EL element is suppressed in decrease of the luminance and its life time is remarkably improved.

## Description

### Technical Field

The present invention relates to an organic EL element and a method for producing the same. More particularly, the present invention relates to a technique which specifies the amount of a remaining precursor compound contained in an organic compound material constituting an emissive layer in an organic EL element to prepare a high-performance organic EL element.

### Background Art

An organic EL element is a light emitting device having a structure in which an organic emissive material is interposed between a cathode and an anode, and is a light emitting device based on the principle that an electron injected from a cathode and a hole injected from an anode recombine in an organic layer and energy generated thereby is drawn out as luminescence energy.

At present, as a method for preparing an organic layer contained in an organic device typified by an organic EL element, dry processes such as a vacuum deposition process and a patterning method using a deposition mask are general. However, there were problems that in the vacuum deposition process, production apparatuses are expensive and that use efficiency of a material is low. Further, in the patterning method using a deposition mask, there was a problem that preparation of a large-area device is difficult.

In contrast, a method for preparing an organic device based on a wet process such as spin coating or ink-jetting has also been investigated. However, most materials for an organic device, which are usually used in the vacuum deposition process, are hardly soluble, and it is difficult to use these materials for the wet process as they are. Therefore, it has been investigated to make the organic device materials soluble. Among these investigations, there is a method of using a material which can be converted to the organic device material and is soluble, a so-called soluble precursor. This is a method in which the soluble precursor is applied to a device substrate to form a film and then the soluble precursor is converted to the organic device material by treatment such as heating (see Patent Documents 1 and 2). In accordance with this method, even though a material is very insoluble, if its precursor is soluble, the wet process becomes applicable.

On the other hand, the organic device has been known to decrease in performance with time in association with driving. The effect of impurities immixed in a material in the organic device is considered as a cause of the fact. For example, it has been disclosed that in an organic EL element in which a specific naphthacene derivative is contained in an organic emissive layer, a reduction in luminance of the organic EL element can be reduced and a lengthened life time of the organic EL element can be realized by reducing the amount of a by-product at the time of synthesizing an organic emissive material or the amount of a diol which is a raw material (see Patent Document 3).

### Citation List

### Patent Documents

Patent Document 1: Japanese Unexamined Patent Publication No. 2003-304014
Patent Document 2: Japanese Unexamined Patent Publication No. 2005-232136
Patent Document 3: Japanese Patent No. 4308317

### Summary of Invention

### Technical Problem

When the above-mentioned soluble precursor is used for the organic EL element, it is possible to produce an element based on the wet process, and preparation of a large-area device becomes easy, but there is a possibility that an unconverted soluble precursor may remain and there is apprehension that performance of the organic EL element may be deteriorated.

It is an object of the present invention to provide an organic EL element produced by using a precursor compound of an emissive material or the like, which is suppressed in the time degradation of luminance and has a lengthened life time. Solution to Problem

The present invention pertains to an organic EL element comprising an organic compound layer including at least an emissive layer interposed between a pair of electrodes, wherein the emissive layer contains a naphthacene derivative represented by the following general formula (1) and a naphthacene precursor compound which is a precursor of the naphthacene derivative and is represented by the following general formula (2) and wherein the amount of the naphthacene precursor compound having a bicyclo structure contained in the emissive layer is 5.0 parts by weight or less with respect to 100 parts by weight of the naphthacene derivative:

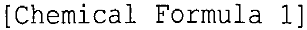

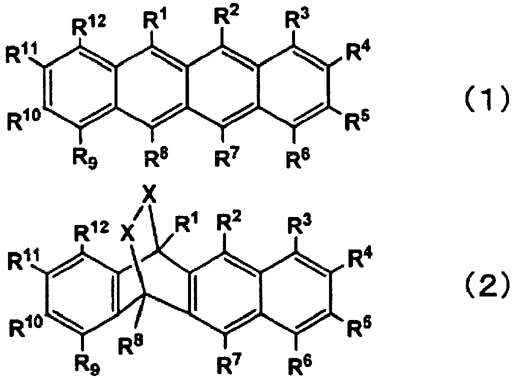

in the general formulas (1) and (2), R¹ to R¹² each may be the same or different and are selected from among hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a cyano group, a carbonyl group, a carboxyl group, an oxycarbonyl group, a carbamoyl group, an amino group, a silyl group, a phosphine oxide group and a condensed ring formed by combining adjacent substituents with each other, and in the general formula (2), X is an atom or an atomic group selected from among C=O, CH₂, O and CHR²¹, and R²¹ is a substituent selected from among an alkyl group, an alkenyl group, an alkoxy group and an acyl group and R²¹s may have a bond therebetween to form a ring. Advantageous Effects of Invention

In accordance with the present invention, the organic EL element is suppressed in decrease of the luminance and its life time is remarkably improved.

### Brief Description of Drawings

Fig. 1 is a sectional view showing an example of an organic EL element in which an emissive layer is patterned according to the present invention.
Fig. 2 is a view showing an example of a shape of an ITO prepared on a glass substrate.

### Mode for Carrying out Invention

The organic EL element of the present invention is an element in which an emissive layer contains a naphthacene derivative and the amount of a naphthacene precursor compound having a bicyclo structure contained in the emissive layer is small.

One of preferred examples of the method for preparing an organic EL element of the present invention is a method of using a wet process. Specifically, a naphthacene precursor compound having a bicyclo structure is used which can be converted to the naphthacene derivative to be used for the organic EL element of the present invention and is soluble. The naphthacene precursor compound having a bicyclo structure has high solubility for many solvents and has a property that it is converted to the naphthacene derivative by a conversion treatment such as light irradiation or heat treatment.

For example, when a thin film of the naphthacene precursor compound having a bicyclo structure is prepared, by a wet process, on the location where the emissive layer is formed in the organic EL element, an emissive layer containing the naphthacene derivative can be prepared by converting the thin film. Also, as another method, there is a method in which the naphthacene precursor compound having a bicyclo structure is applied onto a donor substrate and converted to the naphthacene derivative, and then the naphthacene derivative formed by the conversion treatment is transferred to the location where the emissive layer on the device substrate is to be formed, and by this method, an organic EL element having an emissive layer containing the naphthacene derivative can be prepared.

In the above-mentioned conversion treatment, there may be cases where the naphthacene precursor compound having a bicyclo structure remains without being completely converted to the naphthacene derivative. It has become evident that the amount of the naphthacene precursor compound having a bicyclo structure in the emissive layer is preferably small in order to achieve a lengthened life time of the organic EL element.

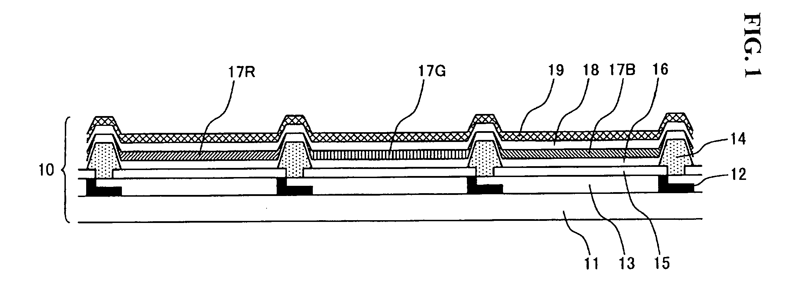

Hereinafter, the organic EL element of the present invention will be described in detail. Fig. 1 is a sectional view showing an example of a typical structure of an organic EL element 10 (display). An active matrix circuit composed of a TFT 12, a planarization layer 13 and the like is configured on a support 11. An element part is composed of a first electrode 15/a hole transporting layer 16/an emissive layer 17/an electron transporting layer 18/a second electrode 19, which are formed on the active matrix circuit. An insulating layer 14 to prevent the occurrence of short-circuit at the end of electrode and define an emissive region is formed at an end of the first electrode. The configuration of the organic EL element is not limited to this example, and for example, only one emissive layer having both a hole transporting function and an electron transporting function may be formed between the first electrode and the second electrode, or the hole transporting layer may have a laminate structure of a multi-layer of a hole injection layer and a hole transporting layer, or the electron transporting layer may have a laminate structure of a multi-layer of an electron transporting layer and an electron injection layer, or the electron transporting layer may be omitted when the emissive layer has an electron transporting function. Moreover, each layer may be laminated in the order of first electrode/electron transporting layer/emissive layer/hole transporting layer/second electrode. Further, any of these layers may be a single-layer or may be a multi-layer. In addition, but not shown, the formation of a protective layer, or the formation and seal of a color filter may be performed using a publicly known technique after the formation of the second electrode.

The emissive layer of the organic EL element of the present invention contains a naphthacene derivative represented by the following general formula (1) and further contains a naphthacene precursor compound having a bicyclo structure, which is a precursor of the naphthacene derivative and is represented by the following general formula (2). In this case, it is important that the amount of the naphthacene precursor compound having a bicyclo structure, which is contained in the emissive layer and represented by the general formula (2), is 5.0 parts by weight or less with respect to 100 parts by weight of the naphthacene derivative represented by the general formula (1). It is more preferably 0.001 parts by weight or more and 5.0 parts by weight or less. The naphthacene derivative represented by the general formula (1) is a compound which exerts a function as an emissive material, particularly, a host compound. In contrast, the naphthacene precursor compound having a bicyclo structure itself is low in function as a host compound. Thus, by setting the amount of the naphthacene precursor compound to 5.0 parts by weight or less with respect to 100 parts by weight of the naphthacene derivative represented by the general formula (1), the host purity in the emissive layer of the organic EL element is improved and thereby the lengthened life time of the element can be achieved. Further, when the host purity is improved, luminance efficiency is improved because a probability that a hole or an electron is trapped in the emissive layer is decreased. More preferably, the amount of the naphthacene precursor compound having a bicyclo structure, which is represented by the general formula (2), is 1.0 part by weight or less with respect to 100 parts by weight of the naphthacene derivative represented by the general formula (1). By setting the amount thereof to 1.0 part by weight or less, the life time further lengthened of the organic EL element can be achieved:

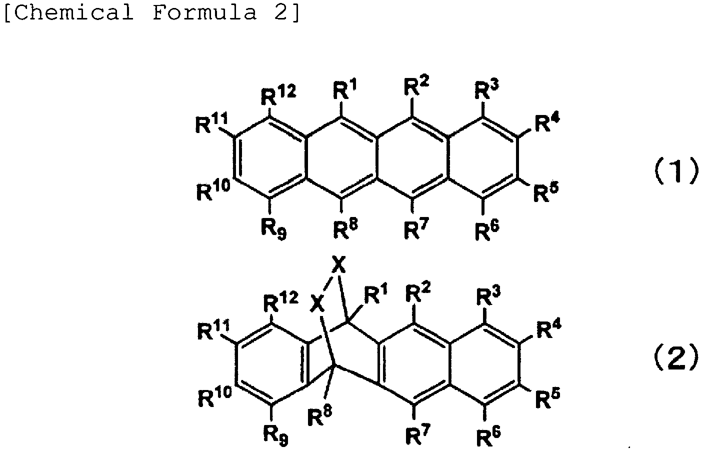

in the general formulas (1) and (2), R¹ to R¹² each may be the same or different and are selected from among hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a cyano group, a carbonyl group, a carboxyl group, an oxycarbonyl group, a carbamoyl group, an amino group, a silyl group, a phosphine oxide group and a condensed ring formed by combining adjacent substituents with each other, and in the general formula (2), X is an atom or an atomic group selected from among C=O, CH₂, O and CHR²¹, and R²¹ is a substituent selected from among an alkyl group, an alkenyl group, an alkoxy group and an acyl group and R²¹s may have a bond therebetween to form a ring.

Among these substituents, the alkyl group represents a saturated aliphatic hydrocarbon group such as a methyl group, an ethyl group, a n-propyl group, an isopropyl group, a n-butyl group, a sec-butyl group or a tert-butyl group, and it may have a substituent or may have no substituent. When substituted, the additional substituent is not particularly limited, and examples thereof include an alkyl group, an aryl group, and a heteroaryl group, and this respect is common to the following description. Further, the number of carbon atoms of the alkyl group is not particularly limited and is usually 1 or more and 20 or less, and more preferably in the range of 1 or more and 8 or less in view of availability and cost.

The cycloalkyl group represents a saturated alicyclic hydrocarbon group such as a cyclopropyl group, a cyclohexyl group, a norbornyl group or an adamantyl group, and it may have a substituent or may have no substituent. The number of carbon atoms of alkyl group portions is not particularly limited and is usually in the range of 3 or more and 20 or less.

The heterocyclic group represents an aliphatic ring having an atom other than carbon in the ring, such as a pyran ring, a piperidine ring or a cyclic amide, and it may have a substituent or may have no substituent. The number of carbon atoms of the heterocyclic group is not particularly limited and is usually in the range of 2 or more and 20 or less.

The alkenyl group represents an unsaturated aliphatic hydrocarbon group containing a double bond, such as a vinyl group, an allyl group or a butadienyl group, and it may have a substituent or may have no substituent. The number of carbon atoms of the alkenyl group is not particularly limited and is usually in the range of 2 or more and 20 or less.

The cycloalkenyl group represents an unsaturated alicyclic hydrocarbon group containing a double bond, such as a cyclopentenyl group, a cyclopentadienyl group or a cyclohexenyl group, and it may have a substituent or may have no substituent. The number of carbon atoms of the cycloalkenyl group is not particularly limited and is usually in the range of 2 or more and 20 or less.

The alkynyl group represents an unsaturated aliphatic hydrocarbon group containing a triple bond, such as an ethynyl group, and it may have a substituent or may have no substituent. The number of carbon atoms of the alkynyl group is not particularly limited and is usually in the range of 2 or more and 20 or less.

The alkoxy group represents a functional group in which an aliphatic hydrocarbon group is bound via an ether group, such as a methoxy group, an ethoxy group or a propoxy group, and this aliphatic hydrocarbon group may have a substituent or may have no substituent. The number of carbon atoms of the alkoxy group is not particularly limited and is usually in the range of 1 or more and 20 or less.

The alkylthio group represents a group formed by substitution of a sulfur atom for the oxygen atom of the ether bond of an alkoxy group. The hydrocarbon group of the alkylthio group may have a substituent or may have no substituent. The number of carbon atoms of the alkylthio group is not particularly limited and is usually in the range of 1 or more and 20 or less.

The aryl ether group represents a functional group in which an aromatic hydrocarbon group is bound via an ether bond, such as a phenoxy group, and the aromatic hydrocarbon group may have a substituent or may have no substituent. The number of carbon atoms of the aryl ether group is not particularly limited and is usually in the range of 6 or more and 40 or less.

The arylthio ether group represents a group formed by substitution of a sulfur atom for the oxygen atom of the ether bond of an aryl ether group. An aromatic hydrocarbon group in the arylthio ether group may have a substituent or may have no substituent. The number of carbon atoms of the arylthio ether group is not particularly limited and is usually in the range of 6 or more and 40 or less.

The aryl group represents an aromatic hydrocarbon group such as a phenyl group, a naphthyl group, a biphenyl group, a fluorenyl group, a phenanthryl group, a terphenyl group, an anthracenyl group or a pyrenyl group, or a group in which a plurality of these groups are linked with one another, and it may be unsubstituted or may be substituted. The number of carbon atoms of the aryl group is not particularly limited and is usually in the range of 6 or more and 40 or less. Examples of the substituent that may be possessed by such an aryl group include an alkyl group, a cycloalkyl group, an alkenyl group, an alkynyl group, an alkoxy group, an aryl ether group, an alkylthio group, halogen, a cyano group, an amino group (the amino group may be further substituted with an aryl group or a heteroaryl group), a silyl group and a boryl group.

The heteroaryl group represents an aromatic group having an atom other than carbon in the ring, such as a furanyl group, a thiophenyl group, an oxazolyl group, a pyridyl group, a quinolinyl group and a carbazolyl group, and it may have a substituent or may have no substituent. The number of carbon atoms of the heteroaryl group is not particularly limited and is usually in the range of 2 or more and 30 or less. The substituent that may be possessed by such a heteroaryl group may be similar to those that may be possessed by the aryl group.
Halogen is fluorine, chlorine, bromine or iodine.

The carbonyl group represents a substituent containing a carbon-oxygen double bond, such as an acyl group or a formyl group. The acyl group represents a substituent formed by substitution of an alkyl group, an aryl group or a heteroaryl group for the hydrogen of a formyl group. The oxycarbonyl group represents a substituent containing an ether bond on the carbon of the carbonyl group, such as a tert-butyloxycarbonyl group or a benzyloxycarbonyl group.

The carbamoyl group represents a substituent obtained by excluding a hydroxyl group from a carbamic acid, and it may have a substituent or may have no substituent. The amino group represents a nitrogen compound group such as a dimethylamino group, and it may be unsubstituted or may be substituted. The silyl group represents a silicon compound group such as a trimethylsilyl group, and it may be unsubstituted or may be substituted. The number of carbon atoms of the silyl group is not particularly limited and is usually in the range of 3 or more and 20 or less. Further, the number of silicon atoms of the silyl group is usually 1 to 6. The phosphine oxide group represents a substituent containing a phosphorus-oxygen double bond, and it may be unsubstituted or may be substituted.

The condensed ring formed by combining adjacent substituents with each other, describing based on the above general formula (1), refers to a conjugated or nonconjugated condensed ring formed when any adj acent two substituents (e.g. , R¹⁰ and R¹¹) selected from among R¹ to R¹² are combined with each other. These condensed rings may contain a nitrogen atom, an oxygen atom or a sulfur atom in a ring structure or may be coupled with another ring.

In the present invention, the naphthacene derivative represented by the general formula (1) more preferably has a structure represented by the following general formula (3) since luminance efficiency is improved. Further, the naphthacene precursor compound having a bicyclo structure more preferably has a structure represented by the following general formula (4) corresponding to the naphthacene derivative represented by the general formula (3):

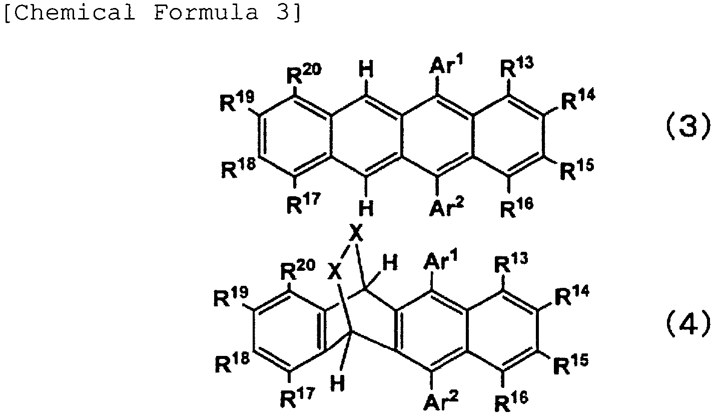

in the general formulas (3) and (4), R¹³ to R²⁰ each may be the same or different and are selected from among hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a cyano group, a carbonyl group, a carboxyl group, an oxycarbonyl group, a carbamoyl group, an amino group, a silyl group, a phosphine oxide group and a condensed ring formed by combining adjacent substituents with each other. Ar¹ and Ar² are selected from among an aryl ether group, an aryl thioether group, an aryl group and a heteroaryl group. Ar¹ and Ar² may have a substituent or may have no substituent. In the general formula (4), X is an atom or an atomic group selected from among C=O, CH₂, O and CHR²². R²² is a substituent selected from among an alkyl group, an alkenyl group, an alkoxy group and an acyl group and R²²s may have a bond therebetween to form a ring.

Herein, descriptions about respective substituents of R¹³ to R²⁰ and Ar¹ and Ar² are the same as those about R¹ to R¹² in the general formula (1).

Herein, R¹³ to R²⁰ are preferably selected from among hydrogen, an alkyl group, a heterocyclic group, an alkoxy group, an alkylthio group, an aryl ether group, an aryl thioether group, an aryl group, a heteroaryl group, and a condensed ring formed by combining adjacent substituents with each other, because high organic EL performance can be exerted. Among these, R¹³ to R²⁰ are particularly preferably selected from among hydrogen, an alkyl group, a heterocyclic group, an aryl group, a heteroaryl group, and a condensed ring formed by combining adjacent substituents with each other.

Further, examples of Ar¹ and Ar² include, but are not particularly limited to, the following. Ar¹ and Ar² may be the same or different.

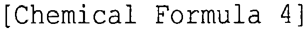

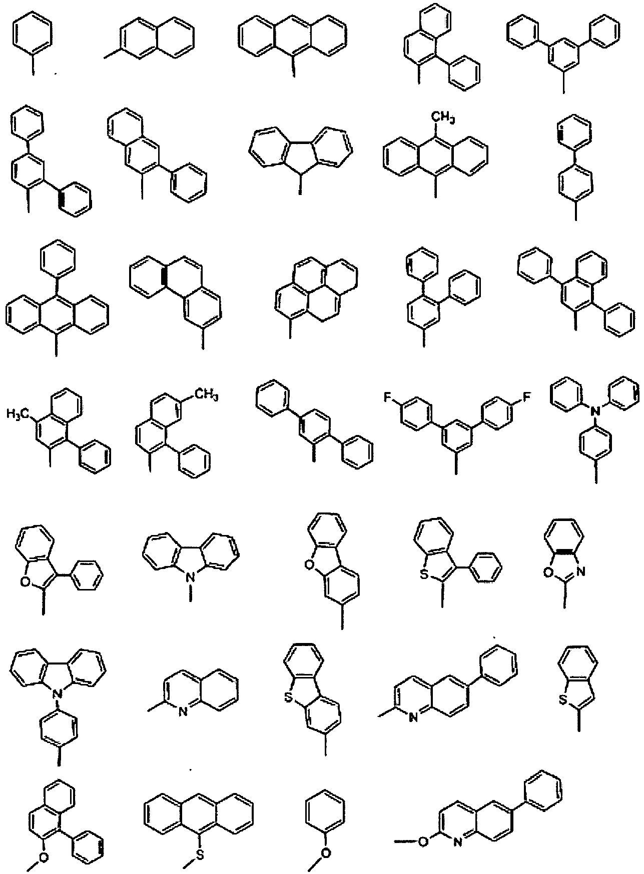

Further, the naphthacene precursor compound having a bicyclo structure more preferably has an ethylene-bridged structure or a diketo-bridged structure, and particularly preferably has a diketo-bridged structure. That is, in the naphthacene precursor compound having a bicyclo structure, which is represented by the general formulas (1) and (3), X is preferably C=O or CH₂, and more preferably C=O. By employing the above structure, the conversion from the naphthacene precursor compound to the naphthacene derivative can be performed more efficiently.

Examples of the naphthacene derivative represented by the general formula (1) or (3) include the following structures.

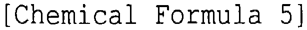

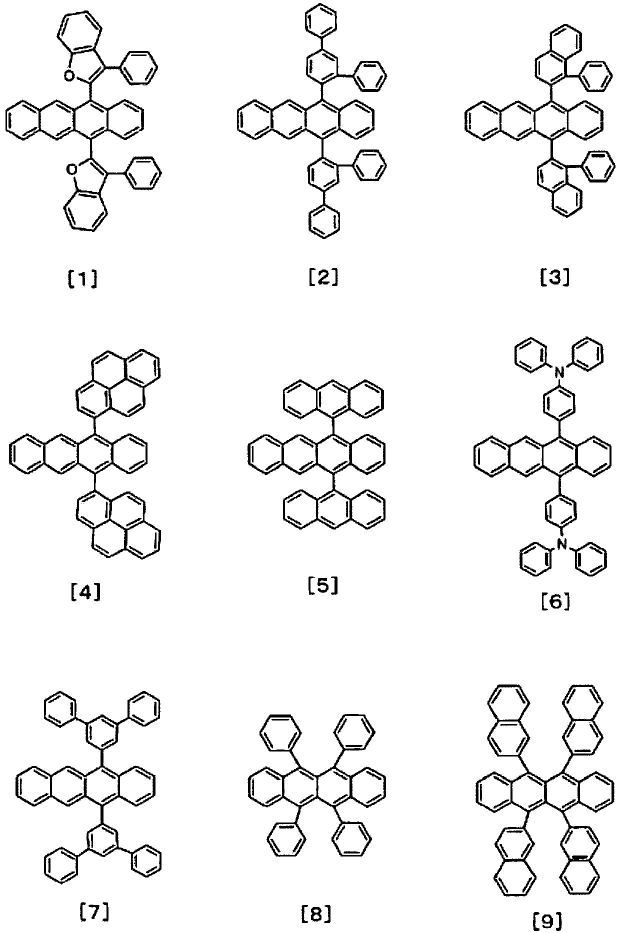

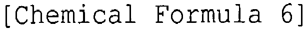

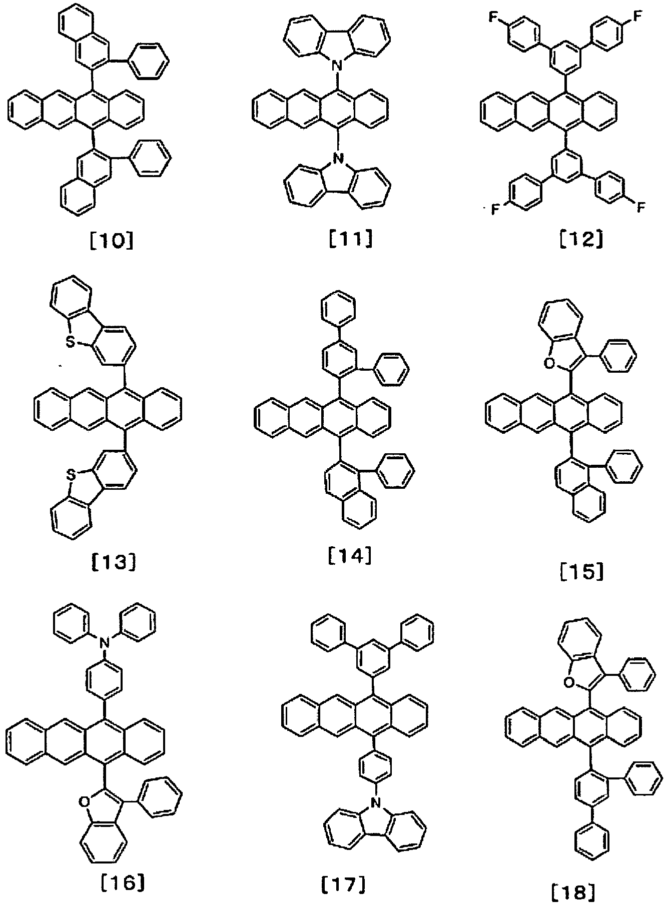

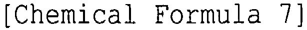

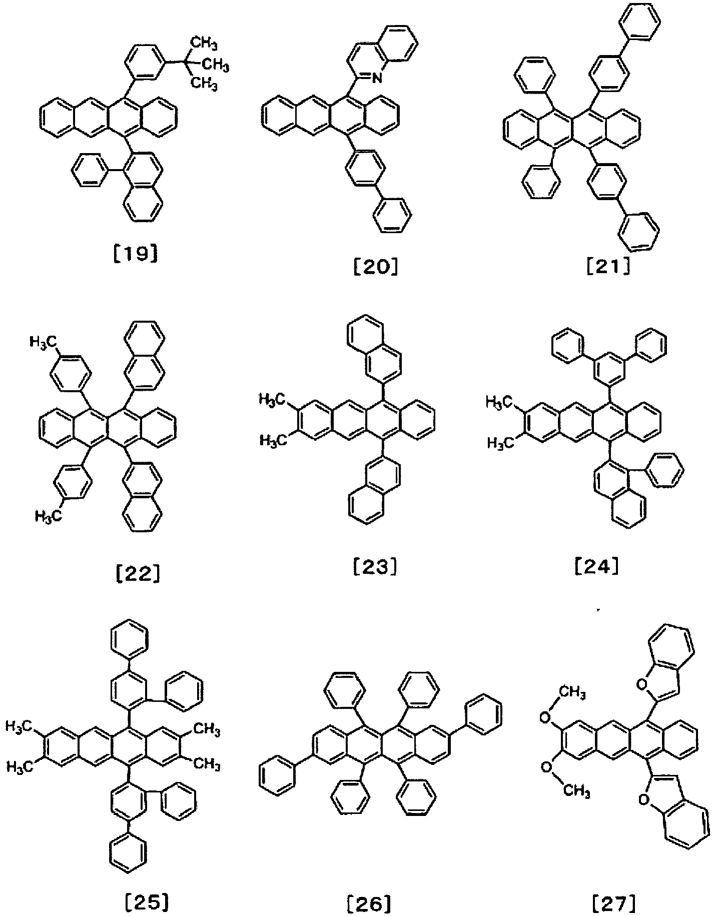

In addition, as described above, since the naphthacene derivative represented by the general formula (1) exhibits a function as a host compound, the present invention can be broadly applied to a compound which can prepare a naphthacene precursor compound having a bicyclo structure among such compounds. Accordingly, the naphthacene derivative represented by the general formula (1) or (3) is not limited to the above compounds, and the naphthacene derivatives other than the above compounds, described in Japanese Patent No. 4308317 (particularly paragraphs 0033 to 0057), International Publication WO 2007/097178 pamphlet (particularly paragraphs 0015 to 0024), Japanese Patent No. 3712760 (particularly paragraphs 0011 to 0039) and the like, can be preferably used. Those having a structural isomer of such naphthacene derivatives can be more preferably used. Among these, disubstituted naphthacene, tetrasubstituted naphthacene, disubstituted pentacene and the like are particularly preferred. Herein, the disubstituted naphthacene is preferably naphthacene in which a fifth position and a twelfth position in a naphthacene skeleton are substituted. Further, the tetrasubstituted naphthacene is preferably naphthacene in which a fifth position, a sixth position, an eleventh position and a twelve position in a naphthacene skeleton are substituted. Also, the disubstituted pentacene is preferably pentacene in which a sixth position and a thirteenth position in a pentacene skeleton are substituted.

Further, when the compound [1] is a naphthacene derivative, examples of the corresponding naphthacene precursor compound having a bicyclo structure include the following structures:

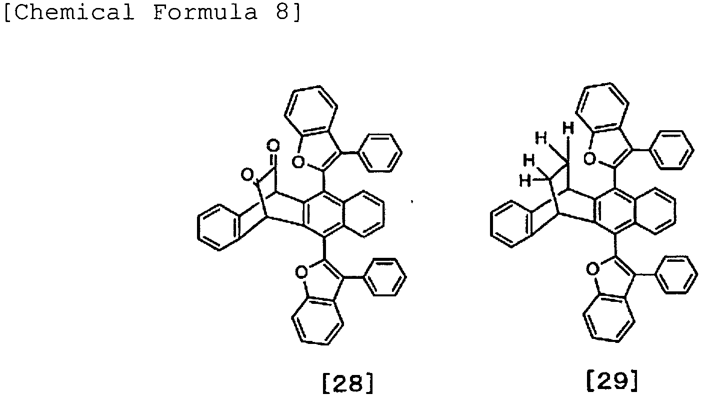

Further, when the compound [2] is a naphthacene derivative, examples of the corresponding naphthacene precursor compound having a bicyclo structure include the following structures:

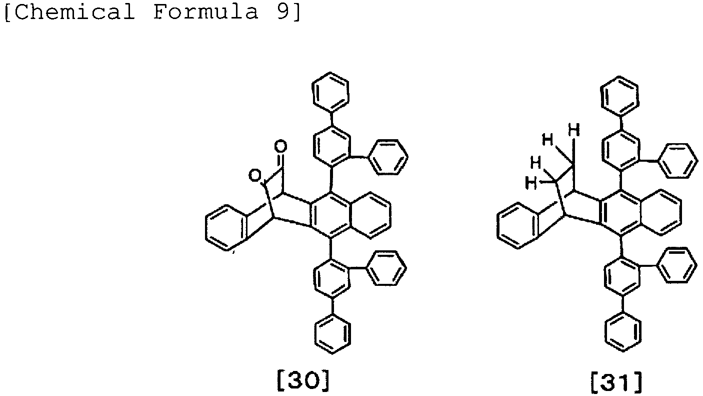

Further, when the compound [3] is a naphthacene derivative, examples of the corresponding naphthacene precursor compound having a bicyclo structure include the following structures:

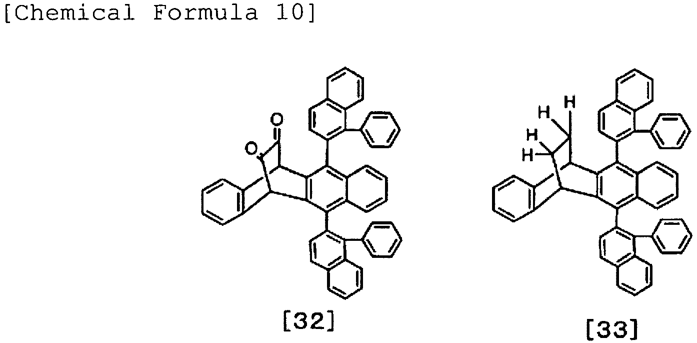

Also concerning other naphthacene derivatives described in the present specification or the above-mentioned documents, the corresponding naphthacene precursor compounds having a bicyclo structure can be shown in the same manner.

Further, the naphthacene derivative and the naphthacene precursor compound having a bicyclo structure used in the present invention can be synthesized by a publicly known method. Examples of a method of synthesizing the naphthacene derivative include a method in which an aryl group is coupled with the fifth position and twelfth position of naphthacene. One example thereof includes a method in which halogenated aryl is coupled with 5, 12-naphthacene quinone by use of n-butyllithium and then the coupled compound is reduced with a tin catalyst. Further, examples of a method of synthesizing the naphthacene precursor compound having a bicyclo structure include a method of using a corresponding naphthacene derivative. For example, when by Diels-Alder reaction, vinylene carbonate is added to the sixth position and eleventh position of the naphthacene derivative in which an aryl group is coupled with the fifth position and twelfth position thereof, and then the resulting compound is hydrolyzed and oxidized by Swern reaction, a bicyclo structure having an diketo-bridged structure can be formed. Moreover, a bicyclo structure having an ethylene-bridged structure can be formed by a method described in Journal of The American Chemical Society, 1965, vol. 87, No. 20, p4649-4651 or the like.

In the naphthacene derivative and the naphthacene precursor compound having a bicyclo structure used in the present invention, it is preferred to eliminate impurities such as a raw material used during the process of synthesis of these naphthacene compounds and a by-product. As a method of eliminating the impurities, for example, a silica gel column chromatography method, a recrystallization method, a reprecipitation method, a filtration method, a sublimation purification method and the like can be used. Two or more of these methods may be used in combination.

Next, the method for producing an organic EL element of the present invention will be described. The organic EL element of the present invention may be produced by a dry process such as a vacuum deposition process, or may be produced by use of a wet process. However, the organic EL element of the present invention is preferably produced by use of the wet process since it is also applicable to the preparation of a large panel in comparison with a conventional vapor-deposition process by a deposition mask. Since the naphthacene precursor compound having a bicyclo structure is soluble, application of the wet process is easy. As a method of the wet process, publicly known methods such as ink-jetting, nozzle printing, spin coating and dipping can be employed.

Herein, the solubility in the present invention specifically means that 0.5 parts by weight or more of the naphthacene precursor material having a bicyclo structure is dissolved in 100 parts by weight of any of the following solvents at room temperature under ordinary pressure. By having the above solubility, an organic thin film having a preferred film thickness as an emissive layer can be prepared by the above-mentioned application method. In addition, it is more preferred that 1.0 part by weight or more thereof is dissolved in the solvent. As the solvent, a general purpose solvent such as toluene, xylene, chlorobenzene, chloroform, dichloromethane, dichloroethane, ethyl acetate, tetrahydrofuran, trimethylbenzene, γ-butyrolactone, n-methylpyrrolidone, tetralin, o-dichlorobenzene, trichlorobenzene and ethyl benzoate can be used, and a solvent having a boiling point and viscosity according to an application method to be used can be selected. These solvent may be used singly or may be used as a mixture of plural-solvents. Further, the naphthacene precursor compound having a bicyclo structure may be dissolved by applying an ultrasonic wave or by carrying out a heat treatment, and a step of filtration may be added after dissolution.

In order to convert the naphthacene precursor compound having a bicyclo structure to the naphthacene derivative, a conversion treatment is carried out regardless of whether the dry process is used or the wet process is used. The conversion treatment referred to herein is treatment of causing changes in structure of the naphthacene precursor compound having a bicycle structure by heating, light-irradiation or contact with chemicals to convert to the intended naphthacene derivative. In this case, as a factor to cause the changes in structure, one, which does not remain within a material constituting the organic EL element, is preferred to avoid a reduction in a characteristic of the organic EL element. Therefore, treatment by heating or light-irradiation or treatment by a volatile compound is preferred. The volatile compound refers to acids or alkalis that do not remain after the treatment, such as a hydrochloric acid ether complex and ammonia gas.

Among these changes in structure, changes in structure by light-irradiation and/or heat treatment is particularly preferred. A hot plate, an inert oven or an infrared heater can be used for heating. When the structure is converted by light-irradiation, it is preferred to use ultraviolet light to visible light. However, since an undesired photoreaction due to ultraviolet light may occur depending on the used precursor compound, visible light is more preferably used. Further, when the structure is converted by contact with chemicals, a method of immersing a substrate in a storage tank containing the chemicals and a method of applying the chemicals by spraying can be exemplified. In any case, the precursor compound may be heated after the contact with the chemicals to promote the conversion. Further, a step of cleaning the chemicals after the conversion may be introduced.

Specific examples of the conversion reaction include a Retro Diels-Alder reaction, a cheletropic reaction, decarboxylation, decarbonylation from a carbonyl compound, and deoxydation. An optimal conversion treatment can be selected for every reaction. For example, when X is C=O in the general formula (2), decarbonylation by light-irradiation is selected, and when X is CH₂, elimination of ethylene by heating is selected.

Further, by appropriately adjusting the time of light-irradiation, the temperature or time of heating, or the kinds or treatment time of the chemicals, it is possible to adjust the amount of the naphthacene precursor compound having a bicyclo structure, which is contained finally in the emissive layer and is represented by the general formula (2), to 5. 0 parts by weight or less and further 1.0 part by weight or less with respect to 100 parts by weight of the naphthacene derivative represented by the general formula (1). For example, lengthening a time for each treatment is generally effective for increasing the amount of conversion and for further reducing the amount of the precursor. Further, light-irradiation under heating has the effect of accelerating a reaction rate depending on the kinds of conversion reaction.

In the case of the reaction by light-irradiation, it is preferred to select light having a wavelength meeting the absorption of the compound, and it is more preferred to select light having a wavelength at which the absorption intensity of the compound is maximum. For example, when X is C=O and decarbonylation by light-irradiation is used, it is preferred to irradiate the compound with light with a wavelength of from 420 nm to 510 nm corresponding to an absorption band originated from a diketo structure. Among these wavelengths, it is more preferred to irradiate the compound with light with a wavelength of from 450 nm to 480 nm at which the absorption intensity is maximum, because the conversion by light can be performed in a short time. Any light source generally known can be used for a light source. Particularly, a light emitting diode can perform the conversion by light efficiently since a desired wavelength can be selected with a relatively narrow half width. Further, when a light source having light with a plurality of wavelengths such as a high-pressure mercury lamp is used, it is desired to cut off the light other than light with a desired wavelength through a blue filter or the like in advance. In this case, the intensity of light is weakened by passing through the filter, but sufficient conversion is possible by increasing an irradiation time.

The specific method for producing an organic EL element of the present invention based on the dry process or the wet process will be described in more detail. In addition, the content of the following description is an example, and the method for producing an organic EL element of the present invention is not limited thereto. First, when the dry process such as a vacuum deposition process is used, the naphthacene precursor compound having a bicyclo structure dissolved in arbitrary solvent is subjected to a conversion treatment, and a naphthacene derivative which becomes insoluble and is precipitated is recovered. By using this, it is possible to prepare an emissive layer by a publicly known method such as a vacuum deposition process. The naphthacene precursor compound having a bicyclo structure may be incorporated into the precipitated naphthacene derivative, but the weight thereof can be adequately reduced by being sufficiently subjected to the conversion treatment. In the above description, a method of subjecting the naphthacene precursor compound having a bicyclo structure in a solution state to the conversion treatment has been described, but the naphthacene precursor compound having a bicyclo structure in a solid state may be subjected to the conversion treatment in place of the above method.

When the wet process is employed, there are two main types of methods. First, a coating solution containing at least a naphthacene derivative compound having a bicyclo structure and a solvent is applied onto a device substrate on which layers up to a hole transporting layer have been formed, and then dried. As the solvent used in this case, a solvent in which a layer to be an underlayer is not dissolved or with which the layer does not react is selected. Thereafter, by subjecting the naphthacene precursor compound to the conversion treatment, it is possible to convert to the naphthacene derivative, and to form an organic layer having high functionality as an emissive layer.

Also, as another method, the naphthacene precursor compound having a bicyclo structure is applied onto a substrate other than the device substrate and converted, and the resulting film is transferred to the device substrate on which layers up to a hole transporting layer have been formed, and thus an organic layer having high functionality as an emissive layer can also be formed. Hereinafter, the above-mentioned substrate other than the device substrate is referred to as a donor substrate.

It is preferred to use the donor substrate because of the following advantages. That is, when an applied film of the naphthacene precursor compound prepared on the donor substrate is subjected to the conversion treatment and then transferred onto the device substrate to form an emissive layer, even if unevenness of application of a material before transferring occurs on the device substrate, the unevenness is resolved during transferring and a uniform device constituent material layer can be formed on the device substrate. In addition, the device constituent material layer is a layer formed of the material contained in the device, and examples thereof include emissive layers (layers formed of a emissive material) in the organic EL element.

As the transferring step, a publicly known method can be employed, and examples of the method include a method in which the donor substrate and the device substrate are opposed to each other and heated from the donor substrate side and a method in which light-irradiation is performed from the donor substrate side. When a structure of the naphthacene precursor compound having a bicyclo structure applied onto the donor substrate is changed by heat, if transfer is performed by way of heating, the remaining naphthacene precursor compound having a bicyclo structure can be more reduced since changes in structure also proceeds during the transfer.

Moreover, further the conversion treatment of the naphthacene precursor compound having a bicyclo structure may be added for the layer transferred onto the device substrate after the transferring step. That is, light-irradiation, heating or chemical treatment may be further applied to the layer transferred onto the device substrate. This makes it possible to further reduce the naphthacene precursor compound having a bicyclo structure, which remains after the conversion treatment on the donor substrate and is transferred together with the naphthacene derivative onto the device substrate. Accordingly, a further lengthened life time of the organic EL element can be achieved.

Further, when the emissive layer is prepared by using the naphthacene precursor compound having a bicyclo structure, the emissive layer may contain a publicly known dopant material. As the dopant material, a publicly known material can be used, and examples thereof include indenoperylene, pyrromethene and derivatives thereof.

The concentration by weight of the naphthacene precursor compound having a bicyclo structure with respect to the naphthacene derivative in the emissive layer in the present invention is a value obtained by analysis through high-performance liquid chromatography-ultraviolet absorptiometry.

Specific analytical conditions in this analysis method will be described. Silica gel is used for a packing material, and particularly silica gel with bonded octadecyl groups, silica gel with bonded octyl groups and silica gel with bonded phenyl groups are suitably used, and these may be selected depending on the kinds of the naphthacene derivative and the naphthacene precursor compound having a bicyclo structure. Acetonitrile, tetrahydrofuran, distilled water, an aqueous phosphoric acid solution, methanol or the like can be used for the mobile phase, and these may be used in combination. Among these, when only acetonitrile, or a mixed solvent of acetonitrile and an aqueous phosphoric acid solution is used, the concentration can be detected with high separability. Further, pressure at which the mobile phase is fed at this time is preferably about 35 to 50 MPa.

Preparation of a portion other than the emissive layer in the organic EL element of the present invention will be described. In addition, the following description is an example and the present invention is not limited thereto. As a preparation example of an organic EL element shown in Fig. 1, layers up to a first electrode 15, that is, a TFT 12, a planarization layer 13 and the first electrode 15 are patterned on a support 11 by a photolithographic process, and then an insulating layer 14 is patterned by a publicly known technique using a photosensitive polyimide precursor material. Thereafter, a hole transporting layer 16 is formed over the entire area by a publicly known technique using a vacuum deposition process. Emissive layers 17R, 17G and 17B are patterned on the hole transporting layer 16 as an underlayer. By forming an electron transporting layer 18 and a second electrode 19 over the entire area thereon by a publicly known technique such as a vacuum deposition process, the organic EL element can be produced.

The emissive layer may be a single-layer or may be a multi-layer as long as it contains the naphthacene derivative represented by the general formula (1), and moreover, the emissive layer may contain other materials. The emissive layer preferably has a single-layer structure of a mixture of a host material and a dopant material from the viewpoints of luminance efficiency and color purity. As a preparation method of the emissive layer, publicly known methods, such as vapor-deposition, solution application, ink-jetting and nozzle printing, can be employed. When a film is formed by the vapor-deposition process, it is preferred to deposit, by vapor-deposition, a material obtained by converting the naphthacene precursor compound having a bicyclo structure of the present invention to the naphthacene derivative to form a film. The naphthacene derivative obtained by converting the naphthacene precursor compound having a bicyclo structure improves the performance of the organic EL element since the naphthacene derivative contains a trans-form structure. Moreover, as described above, particularly preferred is a method in which the applied film of the naphthacene precursor compound having a bicyclo structure prepared on the donor substrate by using a wet process is subjected to the conversion treatment, and thereafter an emissive layer is formed by undergoing the step of transferring onto the device substrate.

The hole transporting layer may be a single-layer or may be a multi-layer, and a material of each layer may be a single material or may be a mixture of a plurality of materials. The hole transporting layer also includes a layer referred to as a hole injection layer. An acceptor material, which promotes a hole transporting property, may be mixed in the hole transporting layer from the viewpoints of a hole transporting property (low driving voltage) and durability. Therefore, a transferring material to form a film of the hole transporting layer may be comprised of a single material, or may be comprised of a mixture of a plurality of materials.

Examples of the hole transporting material include low molecular materials such as aromatic amines typified by N, N'-diphenyl-N, N'-dinaphthyl-1, 1'-diphenyl-4,4'-diamine (NPD), N,N'-biphenyl-N,N'-biphenyl-1, 1'-diphenyl-4,4'-diamine and N,N'-diphenyl-N,N'- (N-phenylcarbazolyl) -1, 1'-diphenyl-4 4'-di amine, and heterocyclic compounds typified by N-isopropylcarbazole, pyrazoline derivatives, stilbene-based compounds, hydrazone-based compounds, oxadiazole derivatives and phthalocyanine derivatives; and polymer materials such as polycarbonate and styrene derivatives having these low molecular materials on the side chains, polyvinylcarbazole and polysilane. Examples of the acceptor material include low molecular materials such as 7,7,8,8-tetracyanoquinodimethane (TCNQ), hexaazatriphenylene (HAT) and cyano group derivatives thereof (HAT-CN6). Further, examples of the hole transporting material or acceptor material also include metal oxides such as molybdenum oxide and silicon oxide which are formed in a small thickness on the surface of the first electrode.

The electron transporting layer may be a single-layer or may be a multi-layer, and a material of each layer may be a single material or may be a mixture of a plurality of materials. The electron transporting layer also includes a layer referred to as a hole blocking layer or an electron injection layer. A donor material, which promotes an electron transporting property, may be mixed in the electron transporting layer from the viewpoints of an electron transporting property (low driving voltage) and durability. The layer referred to as an electron injection layer is often discussed as the donor material. A transferring material to form a film of the electron transporting layer may be comprised of a single material, or may be comprised of a mixture of a plurality of materials.

Examples of the electron transporting material include quinolinol complexes such as Alq₃ and 8-quinolinato lithium (Liq); condensed polycyclic aromatic derivatives such as naphthalene and anthracene; styryl-based aromatic ring derivatives typified by 4,4'-bis(diphenylethenyl)biphenyl; quinone derivatives such as anthraquinone and diphenoquinone; phosphorus oxide derivatives; benzoquinolinol complexes; hydroxyazole complexes; azomethine complexes; various metal complexes such as tropolone metal complexes and flavonol metal complexes; low molecular materials such as compounds having a heteroaryl ring structure including electron-accepting nitrogen; and polymer materials having these low molecular materials on the side chains.
Examples of the donor material include alkali metals and alkaline earth metals such as lithium, cesium, magnesium and calcium; various metal complexes such as quinolinol complexes of alkali metals or alkaline earth metals; and oxides and fluorides of alkali metals or alkaline earth metals such as lithium fluoride and cesium oxide.

At least one of the first electrode and the second electrode is preferably transparent in order to take out light emission from the emissive layer. The first electrode is transparent in the bottom emission in which light is taken out from the first electrode and the second electrode is transparent in the top emission in which light is taken out from the second electrode.

The organic EL element in the present invention is not limited to an active matrix type in which the second electrode is generally formed as a common electrode, and it may be, for example, a passive matrix type including a stripe-like electrode in which the first electrode and the second electrode intersect with each other, or may be a segment type in which a display section is pattered so as to display predetermined information. Examples of the use thereof include a TV set, a personal computer, a monitor, a clock, a thermometer, an audio equipment, and a display panel for automobiles.

### Examples

Hereinafter, the present invention will be described by way of examples, but the present invention is not limited to these examples.

1H-NMR was measured by Superconducting FT-NMR EX-270 (manufactured by JEOL Ltd.) using a deuterated chloroform solution.

Measurement was performed by using silica gel with bonded octyl groups for a packing material and a mixed solution of acetonitrile and aqueous phosphoric acid solution for a mobile phase of a HPLC (manufactured by Shimadzu Corporation).

### Synthesis Example 1 (Synthesis of compound [1])

9.5 g of phenacyl bromide (manufactured by Aldrich), 6.8 g of phenol (manufactured by Wako Pure Chemical Industries, Ltd.) and 13.3 g of potassium carbonate (manufactured by Wako Pure Chemical Industries, Ltd.) were dissolved in 192 mL of acetone, and under a nitrogen flow, the solution was stirred for 3 hours while being heated under reflux. After cooling the reactant to room temperature, 200 mL of water was added, followed by extraction with dichloromethane. The organic layer was washed with water two times, dried with magnesium sulfate, and then the solvent was distilled off with a rotary evaporator. The resulting crude reaction product was purified by silica gel column chromatography to obtain an intermediate 1-1 represented by the following formula:

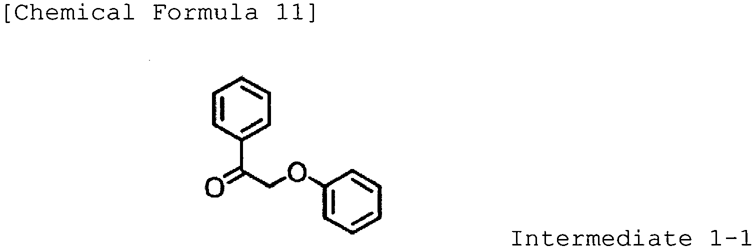

9.7 g of the intermediate 1-1 was dissolved in 200 mL of dichloromethane, and to this, 22.0 g of methanesulfonic acid (manufactured by Tokyo Chemical Industry Co., Ltd.) was added dropwise under a nitrogen flow, and the mixture was stirred for 1 hour while being heated under reflux. After cooling the reactant to room temperature, 200 mL of water was added, followed by extraction with dichloromethane. The organic layer was washed with water two times, dried with magnesium sulfate, and then the solvent was distilled off with a rotary evaporator. The resulting crude reaction product was purified by silica gel column chromatography to obtain 6.9 g of an intermediate 1-2 represented by the following formula:

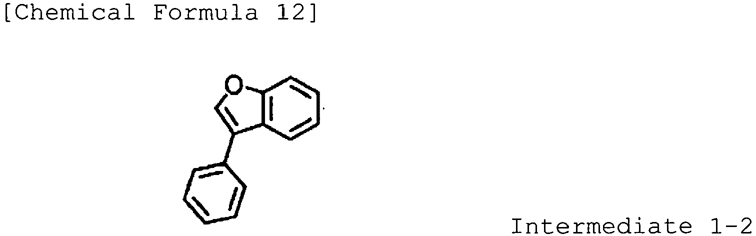

6.0 g of the intermediate 1-2 was dissolved in 124 mL of dehydrated ether, and 23.2 mL of n-butyllithium (1.6M hexane solution) was added dropwise at 0°C under a nitrogen flow and the solution was stirred at 0°C for 1 hour. Next, 20 mL of a dehydrated ether solution containing 2.9 g of 5, 12-naphthacene quinone (manufactured by Tokyo Chemical Industry Co., Ltd.) was added dropwise, and the mixed solution was stirred at room temperature for 1 hour, and then stirred for further 1 hour while being heated under reflux. After cooling the reactant to room temperature, 50 mL of a 0.1 M aqueous hydrochloric acid solution was added, and the mixture was stirred at room temperature for 1 hour, followed by extraction with 250 mL of toluene. The organic layer was washed with water two times, dried with magnesium sulfate, and then the solvent was distilled off with a rotary evaporator. The resulting crude reaction product was dried under vacuum to obtain 6.8 g of an intermediate 1-3 represented by the following formula:

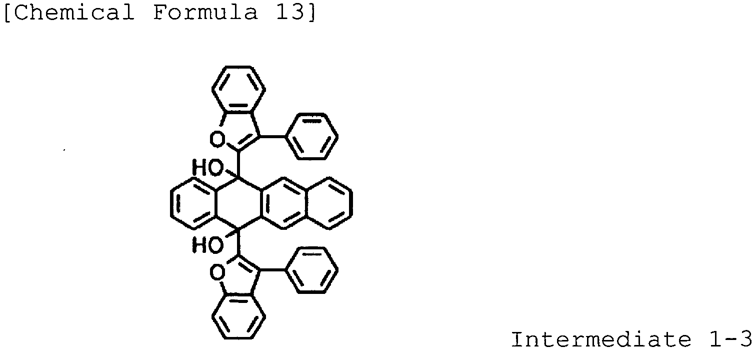

6.8 g of the intermediate 1-3 was dissolved in 210 mL of tetrahydrofuran, and to this, 50 mL of 35% hydrochloric acid solution containing 4.7 g of tin (II) chloride dihydrate (manufactured by Wako Pure Chemical Industries, Ltd.) was added dropwise at room temperature, and the mixture was stirred for 9 hours while being heated under reflux. After cooling the reactant to room temperature, the precipitated powder was collected by filtration and washed with 50 mL of methanol. The resulting powder was stirred for 1 hour in 30 mL of acetone while being heated under reflux, cooled to room temperature, and then the precipitated powder was collected by filtration. Subsequently, the resulting powder was stirred at 60°C for 1 hour in 25 mL of cyclopentyl methyl ether (manufactured by Wako Pure Chemical Industries, Ltd.), cooled to room temperature, and then the precipitated powder was collected by filtration to obtain 3.8 g of a compound [1].
1H-NMR (CDCl₃ (d=ppm)): 7.06-8.29 (m, 26H), 8.50 (s, 2H).

### Synthesis Example 2 (Synthesis of compound [28])

1.0 g of the compound [1] and 2.5 mL of vinylene carbonate (manufactured by Tokyo Chemical Industry Co., Ltd.) were heated under reflux for 10 hours in 16 mL of o-dichlorobenzene (manufactured by Wako Pure Chemical Industries, Ltd.). After cooling the reactant solution to room temperature, 30 mL of hexane was added, and the mixture was stirred. The resulting solution was purified by column chromatography (packing material: silica gel, eluent: hexane/dichloromethane) to obtain 0.89 g of an intermediate 2-1 represented by the following formula:

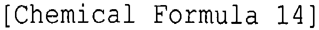

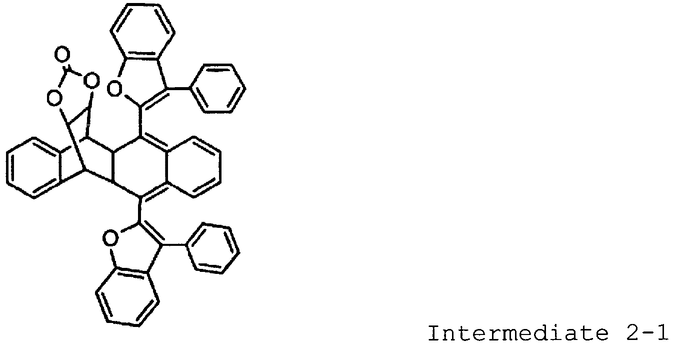

0.89 g of the intermediate 2-1 was dissolved in 30 mL of 1,4-dioxane, and to this, 15 mL of a 4N aqueous sodium hydroxide solution was added in a nitrogen atmosphere, and then the mixed solution was heated under reflux for 4 hours. After completion of the reaction, 30 mL of distilled water and 30 mL of dichloromethane were added and the mixture was stirred. The organic layer was separated, washed with saturated saline and dried with magnesium sulfate. The resulting solution was purified by column chromatography (packing material: silica gel, eluent: hexane/ethyl acetate) to obtain 0.73 g of an intermediate 2-2 represented by the following formula:

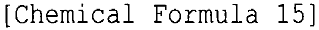

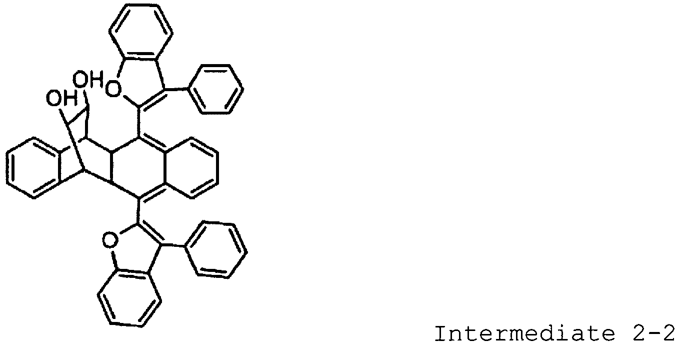

32 mL of dehydrated dichloromethane and 3.2 mL of dehydrated dimethylsulfoxide (manufactured by Aldrich) were cooled to -80°C, and to this, 6.4 mL of trifluoroacetic acid anhydride (manufactured by Wako Pure Chemical Industries, Ltd.) was added dropwise. The mixture was stirred for 20 minutes, and to this, 15 mL of dehydrated dimethylsulfoxide with 0.73 g of the intermediate 2-2 dissolved therein was added dropwise, and the mixture was stirred for 2 hours while maintaining a temperature at -80°C. 16 mL of N,N-diisopropylethylamine (manufactured by Wako Pure Chemical Industries, Ltd.) was gradually added dropwise, and the mixture was stirred for further 2 hours. The temperature of the reactant solution was returned to room temperature, and 25 mL of a 10% aqueous hydrochloric acid solution and 30 mL of dichloromethane were added, and the mixture was stirred for 30 minutes. The organic layer was separated, washed with saturated saline and dried with magnesium sulfate. The resulting solution was concentrated with a rotary evaporator and then purified by column chromatography (packing material: silica gel, eluent: ethyl acetate/toluene) to obtain 250 mg of a compound [28]. ¹H-NMR (CDCl₃ (d=ppm)): 5.02 (s, 2H), 7.00-8.00 (m, 26H).

Synthesis Example 3 (Synthesis of compound [30]) 0.48 g of the compound [2] and 0.1 mL of vinylene carbonate were heated under reflux for 15 hours in 10 mL of o-dichlorobenzene. After cooling the reactant solution to room temperature, a largely excessive amount of hexane was added, and the mixture was vigorously stirred. The resulting powdery solid was filtered and dried to obtain 0. 53 g of an intermediate 3-1 represented by the following formula:

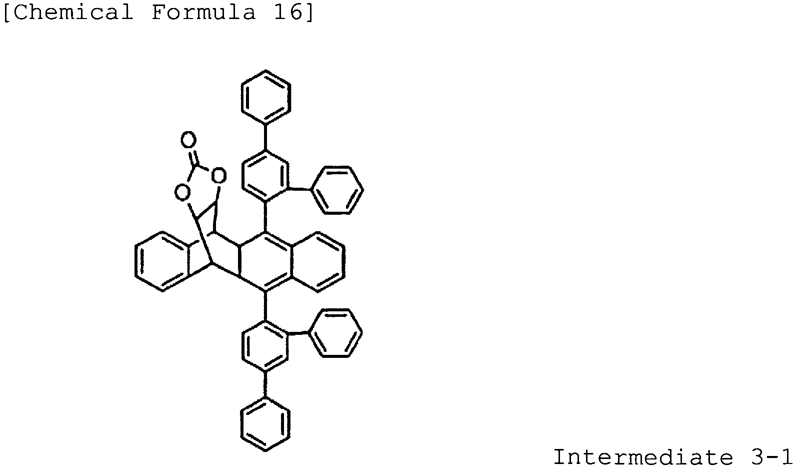

0.33 g of the intermediate 3-1 was dissolved in 20 mL of 1,4-dioxane, and to this, 7.5 mL of a 4N aqueous sodium hydroxide solution was added in a nitrogen atmosphere, and then the mixed solution was heated under reflux for 6 hours. After completion of the reaction, 50 mL of water was added and stirred, and 50 mL of dichloromethane was further added and the mixture was stirred. The organic layer was separated, washed with saturated saline and dried with magnesium sulfate. The dried organic layer was filtered, and then the solvent was condensed and dried to solid to obtain 0.32 g of an intermediate 3-2 represented by the following formula:

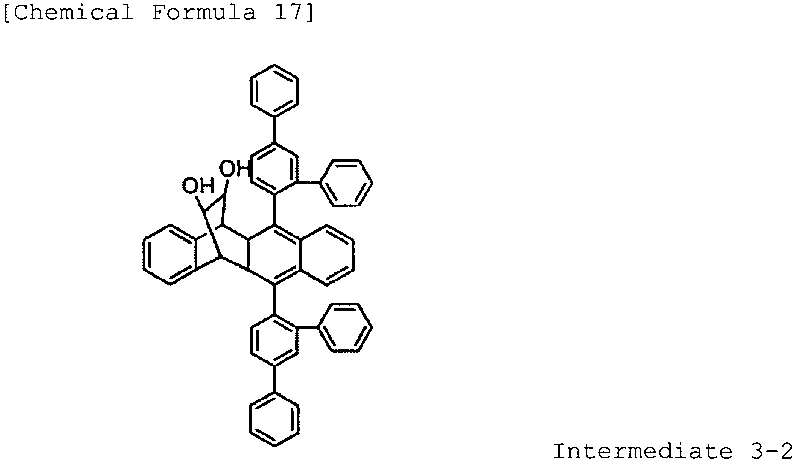

1.2 mL of dimethylsulfoxide was dissolved in 10 mL of dehydrated dichloromethane and the solution was cooled to -78°C. To this, 2.1 mL of acetic acid anhydride was added dropwise, and the mixture was stirred at -78°C for 15 minutes. To the mixture, 10 mL of a dehydrated dichloromethane solution containing 0.25 g of the intermediate 3-2 was gradually added dropwise, and the mixture was stirred at -78°C for 90 minutes. Subsequently, 2.5 mL of triethyl amine was added dropwise, and the mixture was stirred at -78°C for 90 minutes, and then the temperature of the reactant solution was raised to room temperature. After completion of the reaction, dichloromethane was added, and the mixed solution was stirred, and the organic layer was washed with water. The organic layer was separated, dried with magnesium sulfate and filtered, and the filtrate was concentrated to dry. The resulting solid was purified by column chromatography (packing material: silica gel, eluent: dichloromethane) to obtain 0.080 g of a compound [30]. ¹H-NMR (CDCl₃ (d=ppm)): 4.94 (s, 2H), 6.95-7.87 (m, 34H).

Synthesis Example 4 (Synthesis of compound [3]) 10 g of phenylacetylene (manufactured by Tokyo Chemical Industry Co., Ltd.) was dissolved in 200 mL of dehydrated tetrahydrofuran and cooled to 0°C, and 62 mL of n-butyllithium solution (1.6M hexane solution) was added dropwise and the solution was stirred for 1.5 hours. To this, a mixed solution of 6.0 g of phenylacetaldehyde (manufactured by Alfa Aser) and 20 mL of tetrahydrofuran was added dropwise, and the temperature of the mixed solution was raised to room temperature and the mixed solution was stirred for 6 hours. 100 mL of distilled water and 150 mL of ethyl acetate were added to the reactant solution and the solution was stirred. The organic layer was separated, washed with saturated saline and dried with magnesium sulfate. The resulting solution was purified by column chromatography (packing material: silica gel, eluent: hexane/ethyl acetate) to obtain 8.5 g of an intermediate 4-1 represented by the following formula:

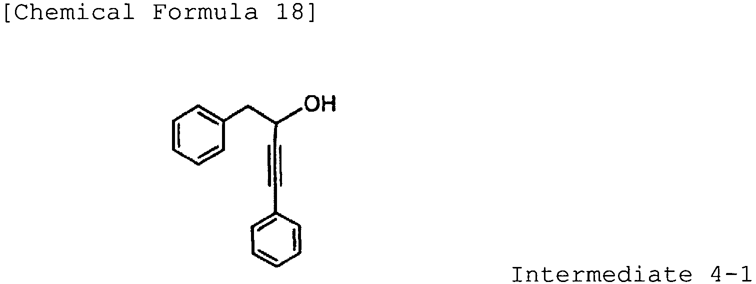

8.5 g of the intermediate 4-1, 6.4 g of sodium hydrogen carbonate (manufactured by Wako Pure Chemical Industries, Ltd.) and 29 g iodine (manufactured by Tokyo Chemical Industry Co., Ltd.) were added to 380 mL of acetonitrile, and the mixture was stirred at room temperature for 4 hours under a nitrogen flow. 150 mL of an aqueous saturated sodium thiosulfate solution and 150 mL of ethyl acetate were added and the mixed solution was stirred. The organic layer was separated, washed with saturated sodium thiosulfate and distilled water, and dried with magnesium sulfate. The resulting solution was purified by column chromatography (packing material: silica gel, eluent: hexane) to obtain 8.7 g of an intermediate 4-2 represented by the following formula:

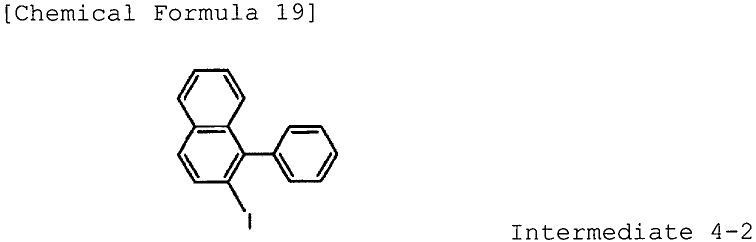

5.3 g of the intermediate 4-2 was dissolved in a mixed solution of 34 mL of toluene and 11 mL of diethyl ether and cooled to -80°C. To this, 10 mL of n-butyllithium solution (1.6M hexane solution) was added dropwise, and the solution was stirred for 3 hours. The temperature of the solution was raised to -40°C, and to this, 1.5 g of 5,12-naphthacene quinone was added, and the mixture was heated to room temperature and stirred for 15 hours. 60 mL of methanol was added to the reactant solution, and the precipitated solid was recovered by filtration to obtain 2.4 g of an intermediate 4-3 represented by the following formula:

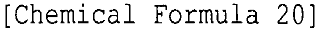

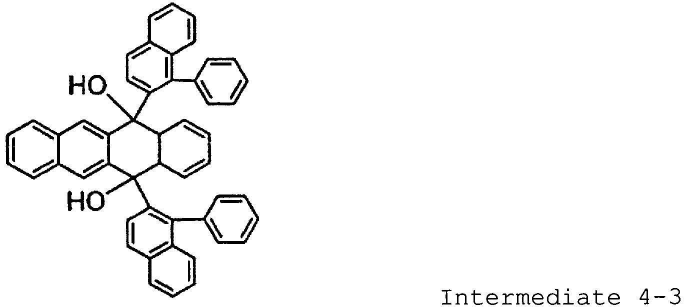

2.4 g of the intermediate 4-3 was added to 36 mL of dried tetrahydrofuran and the temperature of the mixture was raised to 40°C under a nitrogen flow. To this, 19 mL of a 35% hydrochloric acid solution containing 8.14 g of tin (II) chloride dihydrate was added dropwise. After completion of the dropwise addition, the temperature of the mixture was raised to 70°C and the mixture was stirred while being heated under reflux for 4.5 hours. The reactant solution was charged into 150 mL of distilled water, and the precipitated solid was recovered by filtration. Moreover, the solid was washed with distilled water and methanol to obtain 2.3 g of a compound [3] . ¹H-NMR (CDCl₃ (d=ppm)): 6.70-7.74(m, 26H), 8.04-9.09(t, 4H), 8.19(s, 2H).

### Synthesis Example 5 (Synthesis of compound [32])

0.773 g of the compound [3] and 1.73 mL of vinylene carbonate were heated under reflux for 13 hours in 11 mL of o-dichlorobenzene. After cooling the reactant solution to room temperature, 30 mL of hexane was added, and the mixture was stirred. The resulting solution was purified by column chromatography (packing material: silica gel, eluent: hexane/dichloromethane) to obtain 0.92 g of an intermediate 5-1 represented by the following formula:

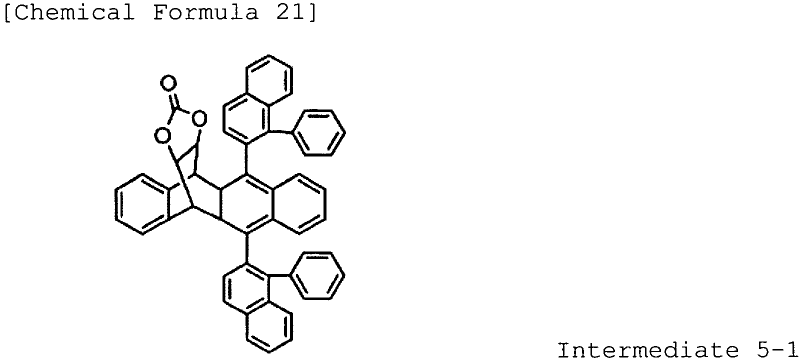

0.92 g of the intermediate 5-1 was dissolved in 28 mL of 1,4-dioxane, and to this, 14 mL of a 4N aqueous sodium hydroxide solution was added in a nitrogen atmosphere, and then the mixed solution was heated under reflux for 4.5 hours. After completion of the reaction, 50 mL of water was added and stirred, and 50 mL of dichloromethane was further added and the mixture was stirred. The organic layer was separated, washed with saturated saline and dried with magnesium sulfate. The resulting solution was purified by column chromatography (packing material: silica gel, eluent: ethyl acetate) to obtain 0.77 g of an intermediate 5-2 represented by the following formula:

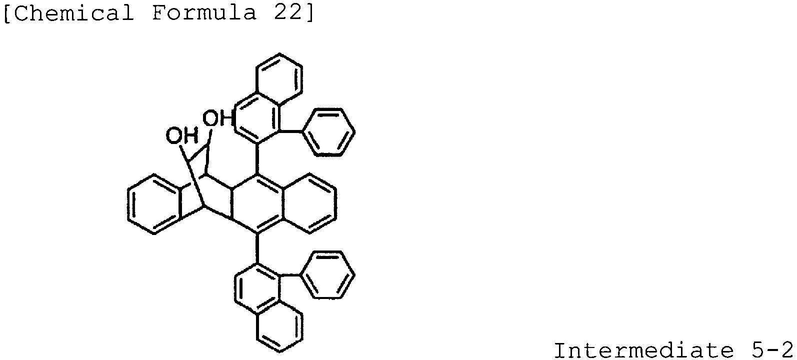

32 mL of dehydrated dichloromethane and 3.2 mL of dehydrated dimethylsulfoxide were cooled to -80°C, and to this, 4.3 mL of trifluoroacetic acid anhydride was added dropwise. The mixture was stirred for 20 minutes, and to this, 15 mL of dehydrated dimethylsulfoxide with 0.75 g of the intermediate 5-2 dissolved therein was added dropwise, and the mixture was stirred for 2 hours while maintaining the temperature at -80°C. 16 mL of N,N-diisopropylethylamine was gradually added dropwise, and the mixture was stirred for further 3 hours. The temperature of the reactant solution was returned to room temperature, and 24 mL of a 10% aqueous hydrochloric acid solution and 30 mL of dichloromethane were added, and the mixture was stirred for 30 minutes. The organic layer was separated, washed with saturated saline and dried with magnesium sulfate. The resulting solution was concentrated with a rotary evaporator and then purified by column chromatography (packing material: silica gel, eluent: ethyl acetate/toluene) to obtain 400 mg of a compound [32].
¹H-NMR (CDCl₃ (d=ppm)): 5. 15 (s, 2H), 6.93-7.68 (m, 30H).

### Example 1

A donor substrate was prepared as described below. An alkali-free glass substrate was used as a support and cleaned/treated in the atmosphere of UV-ozone, and then a titanium layer of 1. 0 µm in thickness was formed over the entire area of the substrate as a light-to-heat conversion layer (LHCL) by a sputtering process. Next, after the LHCL was treated in the atmosphere of UV-ozone, a positive type polyimide-based photosensitive coating agent (manufactured by TORAY INDUSTRIES, INC., DL-1000) was applied onto the LHCL by spin coating, prebaked and exposed to ultraviolet light, and then the exposed area was dissolved in a developer (manufactured by TORAY INDUSTRIES, INC., ELM-D) and eliminated. The polyimide precursor film thus patterned was baked at 350°C for 10 minutes with a hot plate to form a polyimide-based division pattern. The division pattern had a thickness of 2 µm and a forward tapered cross-section. Apertures of 80 µm in width and 280 µm in length through which the LHCL was exposed were arranged with 100-µm pitches in the width direction and 300-µm pitches in the length direction within the division pattern. A chloroform solution containing the compound [28] in an amount of 3% by weight and DCJTB represented by the following formula in an amount of 0.15% by weight was applied onto this substrate by spin coating and dried. The resulting thin film was irradiated with light of a blue-color light emitting diode (central wavelength: 460 nm, half width: 20 nm) for 3 hours in a nitrogen atmosphere to convert the compound [28] to the compound [1]. As a result, a layer including the compound [1], the compound [28] and the DCJTB and having an average thickness of 25 nm was formed within the division pattern (aperture).

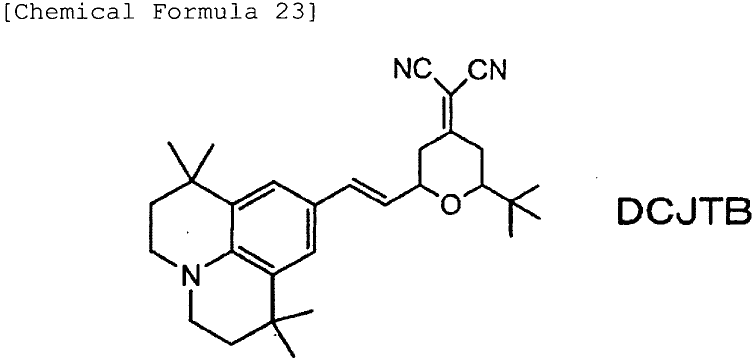

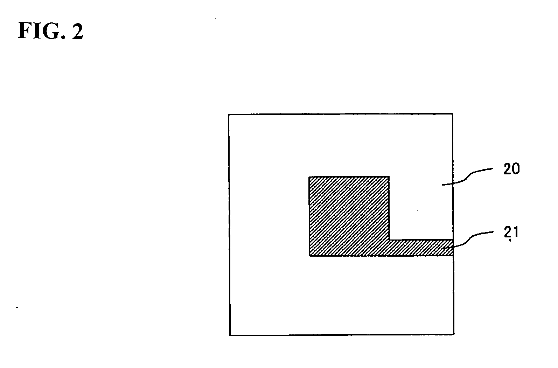

A device substrate was prepared as described below. An alkali-free glass substrate (manufactured by GEOMATEC Co. , Ltd. , article on which a film is formed by sputtering), on which an ITO transparent conductive film of 140 nm in thickness was deposited, was cut into pieces having a size of 38 mm x 46 mm and the ITO film was etched into a shape shown in Fig. 2 by a photolithographic process. Then, a polyimide precursor film patterned in the same manner as in the donor substrate was baked at 300°C for 10 minutes to form a polyimide-based insulating layer. The insulating layer had a height of 1. 8 µm and a forward tapered cross-section. Apertures of 70 µm in width and 270 µm in length through which the ITO was exposed were arranged with 100-µm pitches in the width direction and 300-µm pitches in the length direction within the pattern of the insulating layer. The substrate was treated in the atmosphere of UV-ozone and then placed in a vacuum deposition apparatus and the apparatus was evacuated until the vacuum in the apparatus reached 3 x 10⁻⁴ Pa or less. Copper phthalocyanine (CuPc) was laminated in a thickness of 20 nm and then NPD was laminated in a thickness of 40 nm as a hole transporting layer over the entire area of an emissive region by vapor deposition using a resistance-heating evaporation process.

Next, the position of the division pattern of the donor substrate was aligned opposite to the position of the insulating layer of the device substrate, and these substrates were held in a vacuum of 3 x 10⁻⁴ Pa or less and then taken out to the atmosphere. A transfer space defined by the insulating layer and the division pattern was held at vacuum. In this state, the glass substrate side of the donor substrate was irradiated with light with a central wavelength of 940 nm, of which an irradiation shape was formed into a rectangle of 340 µm in width and 50 µm in length so that a part of materials within the division pattern and a part of the division pattern were simultaneously heated to transfer the materials within the division pattern on a hole transporting layer which was an underlayer of the device substrate. The laser intensity was 148 W/mm², a scanning rate was 0. 6 m/s, and scanning was repeated 24 times by a manner in which laser light overlaps one another so as to transfer the materials to the entire area of an emissive region.

The device substrate to which the materials were previously transferred was placed in a vacuum deposition apparatus again and the apparatus was evacuated until the vacuum in the apparatus reached 3 x 10⁻⁴ Pa or less. E-1 shown below was vapor-deposited in a thickness of 25 nm as an electron transporting layer over the entire area of an emissive region by a resistance-heating evaporation process. Next, lithium fluoride was vapor-deposited in a thickness of 0.5 nm as a donor material (electron injection layer) and further aluminum was vapor-deposited in a thickness of 100 nm as a second electrode to prepare an organic EL element having an emissive region of 5 mm square. In this time, the amount of the compound [28] was measured by HPLC, and consequently it was 2.0 parts by weight with respect to 100 parts by weight of the compound [1].

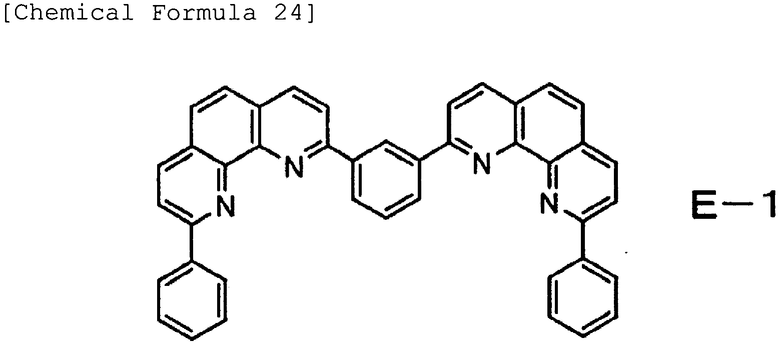

### Example 2

An organic EL element was prepared in the same manner as in Example 1 except for using a compound [34] shown in the following formula in place of the DCJTB. The amount of the compound [28] in the organic EL element prepared in this time was 1.8 parts by weight with respect to 100 parts by weight of the compound [1].

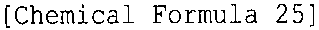

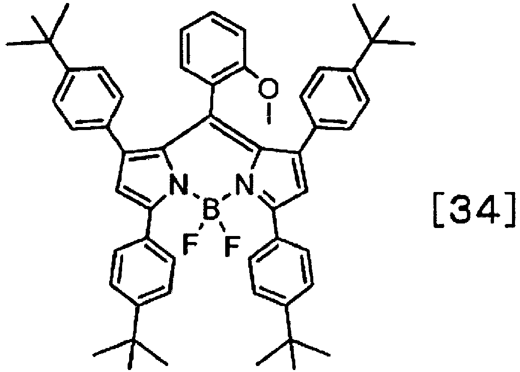

### Example 3

An organic EL element was prepared in the same manner as in Example 1 except for changing the light-irradiation time by the blue-color light emitting diode to 10 hours. The amount of the compound [28] in the organic EL element prepared in this time was 0.3 parts by weight with respect to 100 parts by weight of the compound [1].

### Example 4

An organic EL element was prepared in the same manner as in Example 1 except for using the compound [30] in place of the compound [28]. When the compound [30] is irradiated with the light of the blue-color light emitting diode (central wavelength: 460 nm, half width: 20 nm) for 3 hours, the compound [30] is converted to the compound [2]. The amount of the compound [30] in the organic EL element prepared in this time was 3.1 parts by weight with respect to 100 parts by weight of the compound [2].

### Example 5

An organic EL element was prepared in the same manner as in Example 1 except for using the compound [32] in place of the compound [28]. When the compound [32] is irradiated with the light of the blue-color light emitting diode (central wavelength: 460 nm, half width: 20 nm) for 3 hours, the compound [32] is converted to the compound [3]. The amount of the compound [32] in the organic EL element prepared in this time was 1.5 parts by weight with respect to 100 parts by weight of the compound [3].

### Example 6

An organic EL element was prepared in the same manner as in Example 3 except for irradiating the device substrate, to which the emissive layer was transferred after the transferring step, with the light of the blue-color light emitting diode for 1 hour under vacuum. The amount of the compound [28] in the organic EL element prepared in this time was 0.1 parts by weight with respect to 100 parts by weight of the compound [1].

### Example 7

An organic EL element was prepared in the same manner as in Example 1 except for changing the light-irradiation time by the blue-color light emitting diode to 4 hours. The amount of the compound [28] in the organic EL element prepared in this time was 1.2 parts by weight with respect to 100 parts by weight of the compound [1].

### Example 8

An organic EL element was prepared in the same manner as in Example 1 except for changing the light-irradiation time by the blue-color light emitting diode to 2.5 hours. The amount of the compound [28] in the organic EL element prepared in this time was 2. 6 parts by weight with respect to 100 parts by weight of the compound [1].

### Example 9

An organic EL element was prepared in the same manner as in Example 1 except for changing the light-irradiation time by the blue-color light emitting diode to 2 hours. The amount of the compound [28] in the organic EL element prepared in this time was 4.2 parts by weight with respect to 100 parts by weight of the compound [1].

### Example 10

An organic EL element was prepared in the same manner as in Example 1 except for changing the light-irradiation time by the blue-color light emitting diode to 1.7 hours. The amount of the compound [28] in the organic EL element prepared in this time was 4.8 parts by weight with respect to 100 parts by weight of the compound [1].

### Comparative Example 1

An organic EL element was prepared in the same manner as in Example 1 except for changing the light-irradiation time by the blue-color light emitting diode to 1 hour. The amount of the compound [28] in the organic EL element prepared in this time was 6.3 parts by weight with respect to 100 parts by weight of the compound [1].

### Comparative Example 2

An organic EL element was prepared in the same manner as in Example 1 except for changing the light-irradiation time by the blue-color light emitting diode to 1.5 hours. The amount of the compound [28] in the organic EL element prepared in this time was 5.3 parts by weight with respect to 100 parts by weight of the compound [1].

### Comparative Example 3

An organic EL element was prepared in the same manner as in Example 1 except for using light (peak wavelength 405 nm (half width 5 nm), 436 nm (half width 5 nm)) of a high-pressure mercury lamp (600 W), having passed through a blue filter, in place of the blue-color light emitting diode (central wavelength 460 nm, half width 20 nm). The amount of the compound [28] in the organic EL element prepared in this time was 6.0 parts by weight with respect to 100 parts by weight of the compound [1].

### Example 11

An organic EL element was prepared in the same manner as in Comparative Example 3 except for changing the light-irradiation time through a blue filter by the high-pressure mercury lamp to 7 hours. The amount of the compound [28] in the organic EL element prepared in this time was 4.0 parts by weight with respect to 100 parts by weight of the compound [1].

### Example 12

An organic EL element was prepared in the same manner as in Example 1 except for using the compound [32] in place of the compound [28] and the compound [34] in place of the DCJTB. The amount of the compound [32] in the organic EL element prepared in this time was 1.5 parts by weight with respect to 100 parts by weight of the compound [3].

### Example 13

An organic EL element was prepared in the same manner as in Example 11 except for changing the light-irradiation time by the blue-color light emitting diode to 10 hours. The amount of the compound [32] in the organic EL element prepared in this time was 0.1 parts by weight with respect to 100 parts by weight of the compound [3].

### Example 14

An organic EL element was prepared in the same manner as in Example 11 except for changing the light-irradiation time by the blue-color light emitting diode to 8 hours. The amount of the compound [32] in the organic EL element prepared in this time was 0.3 parts by weight with respect to 100 parts by weight of the compound [3].

### Example 15

An organic EL element was prepared in the same manner as in Example 11 except for changing the light-irradiation time by the blue-color light emitting diode to 5 hours. The amount of the compound [32] in the organic EL element prepared in this time was 0. 6 parts by weight with respect to 100 parts by weight of the compound [3].

### Example 16

An organic EL element was prepared in the same manner as in Example 11 except for changing the light-irradiation time by the blue-color light emitting diode to 2 hours. The amount of the compound [32] in the organic EL element prepared in this time was 2.3 parts by weight with respect to 100 parts by weight of the compound [3].

### Example 17

An organic EL element was prepared in the same manner as in Example 11 except for changing the light-irradiation time by the blue-color light emitting diode to 1 hour. The amount of the compound [32] in the organic EL element prepared in this time was 3.3 parts by weight with respect to 100 parts by weight of the compound [3].

### Example 18

An organic EL element was prepared in the same manner as in Example 11 except for changing the light-irradiation time by the blue-color light emitting diode to 30 minutes. The amount of the compound [32] in the organic EL element prepared in this time was 4.6 parts by weight with respect to 100 parts by weight of the compound [3].

### Comparative Example 4

An organic EL element was prepared in the same manner as in Example 11 except for changing the light-irradiation time by the blue-color light emitting diode to 20 minutes. The amount of the compound [32] in the organic EL element prepared in this time was 6.0 parts by weight with respect to 100 parts by weight of the compound [3].

### Comparative Example 5

An organic EL element was prepared in the same manner as in Example 11 except for changing the light-irradiation time by the blue-color light emitting diode to 10 minutes. The amount of the compound [32] in the organic EL element prepared in this time was 12 parts by weight with respect to 100 parts by weight of the compound [3].

The organic EL elements prepared in Examples 1 to 18 and Comparative Examples 1 to 5 were sealed, and then a constant electric current of 2.5 mA/cm² was passed. The luminance right after starting passing an electric current was measured as initial luminance, and the time that elapsed before the luminance dropped from the initial luminance to one-half under a constant current was measured as a half-life of luminance. Relative values of the measurements in Examples 2 to 11 and Comparative Examples 1 to 3 in the case where the measurement in Example 1 was taken as 1.0 were respectively measured as a relative initial luminance efficiency and as a relative half-life of luminance, and the results are shown in Tables 1 and 2. Further, relative values of the measurements in Examples 13 to 18 and Comparative Examples 4 and 5 in the case where the measurement in Example 12 was taken as 1.0 were respectively measured as a relative initial luminance efficiency and as a relative half-life of luminance, and the results are shown in Tables 3 and 4. Particularly in the life time, there were remarkable differences in performance between both sides of a limit within which the amount of the compound [28] or the compound [32] in the organic EL element was 5.0 parts by weight with respect to 100 parts by weight of the compound [1] or the compound [3].

**[Table 1]**

| | Example 1 | Example 2 | Example 3 | Example 4 | Example 5 | Example 6 | Example 7 |
|---|---|---|---|---|---|---|---|
| Host material | Compound 1 | Compound 1 | Compound 1 | Compound 2 | Compound 3 | Compound 1 | Compound 1 |
| Dopant material | DCJTB | Compound 34 | DCJTB | DCJTB | DCJTB | DCJTB | DCJTB |
| Amount of precursor | 2.0 parts by weight | 1.8 parts by weight | 0.3 parts by weight | 3.1 parts by weight | 1.5 parts by weight | 0.1 parts by weight | 1.2 parts by weight |
| Relative initial luminance efficiency | 1.0 | 1.4 | 1.2 | 1.1 | 1.1 | 1.3 | 1.1 |
| Relative half-life of luminance | 1.0 | 1.1 | 1.3 | 1.0 | 1.1 | 1.5 | 1.0 |

**[Table 2]**

| | Example 8 | Example 9 | Example 10 | Comparative Example 1 | Comparative Example 2 | Comparative Example 3 | Example 11 |
|---|---|---|---|---|---|---|---|
| Host material | Compound 1 | Compound 1 | Compound 1 | Compound 1 | Compound 1 | Compound 1 | Compound 1 |
| Dopant material | DCJTB | DCJTB | DCJTB | DCJTB | DCJTB | DCJTB | DCJTB |
| Amount of precursor | 2.6 parts by weight | 4.2 parts by weight | 4.8 parts by weight | 6.3 parts by weight | 5. 3 parts by weight | 6.0 parts by weight | 4.0 parts by weight |
| Relative initial luminance efficiency | 1.0 | 0.9 | 0.9 | 0.4 | 0.8 | 0.5 | 0.9 |
| Relative half-life of luminance | 0.9 | 0.8 | 0.8 | 0.2 | 0.4 | 0.3 | 0.8 |

**[Table 3]**

| | Example 12 | Example 13 | Example 14 | Example 15 | Example 16 |
|---|---|---|---|---|---|
| Host material | Compound 3 | Compound 3 | Compound 3 | Compound 3 | Compound 3 |
| Dopant material | Compound 34 | Compound 34 | Compound 34 | Compound 34 | Compound 34 |
| Amount of precursor | 1.5 parts by weight | 0.1 parts by weight | 0.3parts by weight | 0.6 parts by weight | 2.3 parts by weight |
| Relative initial luminance efficiency | 1.0 | 1.3 | 1.2 | 1.1 | 1.0 |
| Relative half-life of luminance | 1.0 | 1.4 | 1.3 | 1.3 | 1.0 |

**[Table 4]**

| | Example 17 | Example 18 | Comparative Example 4 | Comparative Example 5 |
|---|---|---|---|---|
| Host material | Compound 3 | Compound 3 | Compound 3 | Compound 3 |
| Dopant material | Compound 34 | Compound 34 | Compound 34 | Compound 34 |
| Amount of precursor | 3.3 parts by weight | 4.6 parts by weight | 6.0 parts by weight | 12 parts by weight |
| Relative initial luminance efficiency | 0.9 | 0.9 | 0.6 | 0.1 |
| Relative half-life of luminance | 1.0 | 0.8 | 0.3 | 0.1 |

### Reference Signs List

- 10: organic EL element (device substrate)
- 11: support
- 12: TFT (including lead-out electrode)
- 13: planarization layer
- 14: insulating layer
- 15: first electrode
- 16: hole transporting layer
- 17: emissive layer
- 18: electron transporting layer
- 19: second electrode
- 20: glass electrode
- 21: ITO pattern

## Claims

1. An organic EL element comprising an organic compound layer including at least an emissive layer interposed between a pair of electrodes, wherein the emissive layer contains a naphthacene derivative represented by the following general formula (1) and the amount of a naphthacene precursor compound present in the emissive layer and represented by the following general formula (2) is 5.0 parts by weight or less with respect to 100 parts by weight of the naphthacene derivative represented by the general formula (1): 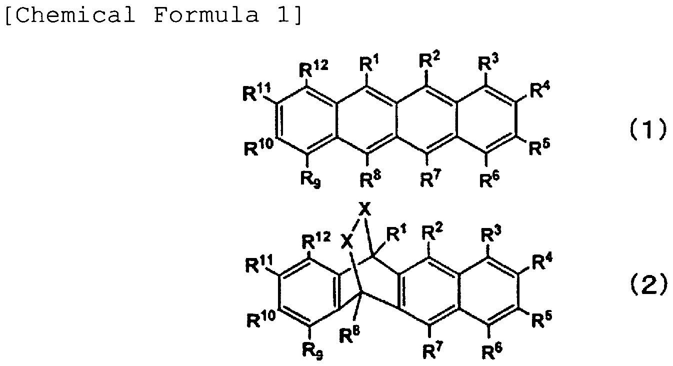 in the general formulas (1) and (2), R¹ to R¹² each may be the same or different and are selected from among hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a cyano group, a carbonyl group, a carboxyl group, an oxycarbonyl group, a carbamoyl group, an amino group, a silyl group, a phosphine oxide group and a condensed ring formed by combining adjacent substituents with each other, and in the general formula (2), X is an atom or an atomic group selected from among C=O, CH₂, O and CHR²¹, and R²¹ is a substituent selected from among an alkyl group, an alkenyl group, an alkoxy group and an acyl group and R²¹s may have a bond therebetween to form a ring.

2. The organic EL element according to claim 1, wherein the amount of the naphthacene precursor compound present in the emissive layer and represented by the general formula (2) is 1.0 part by weight or less with respect to 100 parts by weight of the naphthacene derivative represented by the general formula (1).

3. The organic EL element according to claim 1 or 2, wherein the amount of the naphthacene precursor compound present in the emissive layer and represented by the general formula (2) is 0. 001 parts by weight or more with respect to 100 parts by weight of the naphthacene derivative represented by the general formula (1).

4. The organic EL element according to any one of claims 1 to 3, wherein the naphthacene derivative is represented by the following general formula (3) and the naphthacene precursor compound having a bicyclo structure is represented by the following general formula (4): 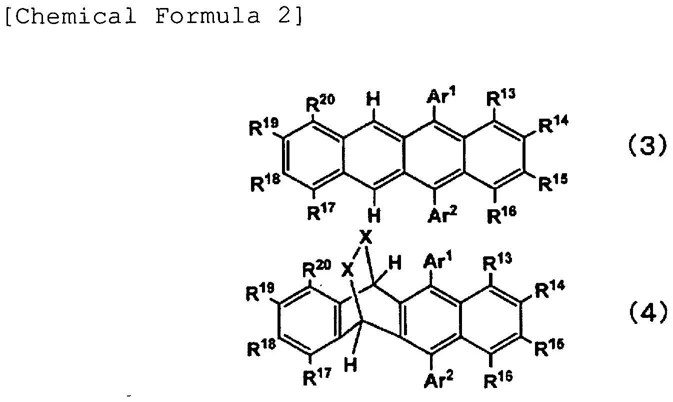 in the general formulas (3) and (4), R¹³ to R²⁰ each may be the same or different and are selected from among hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a cyano group, a carbonyl group, a carboxyl group, an oxycarbonyl group, a carbamoyl group, an amino group, a silyl group, a phosphine oxide group and a condensed ring formed by combining adjacent substituents with each other. Ar¹ and Ar² are selected from the group consisting of an aryl ether group, an aryl thioether group, an aryl group and a heteroaryl group. Ar¹ and Ar² may have a substituent or may have no substituent. In the general formula (4), X is an atom or an atomic group selected from among C=O, CH₂, O and CHR²². R²² is a substituent selected from among an alkyl group, an alkenyl group, an alkoxy group and an acyl group and R²²s may have a bond therebetween to form a ring.

5. The organic EL element according to any one of claims 1 to 4, wherein X is C=O or CH₂ in the naphthacene precursor compound represented by the general formula (2).

6. A method for producing an organic EL element, comprising converting a naphthacene precursor compound represented by the following general formula (2) to a naphthacene derivative represented by the following general formula (1) by conversion treatment, and using a layer including the converted material as an emissive layer of the organic EL element, wherein the amount of the naphthacene precursor compound present in the emissive layer and represented by the general formula (2) is 5.0 parts by weight or less with respect to 100 parts by weight of the naphthacene derivative represented by the general formula (1): 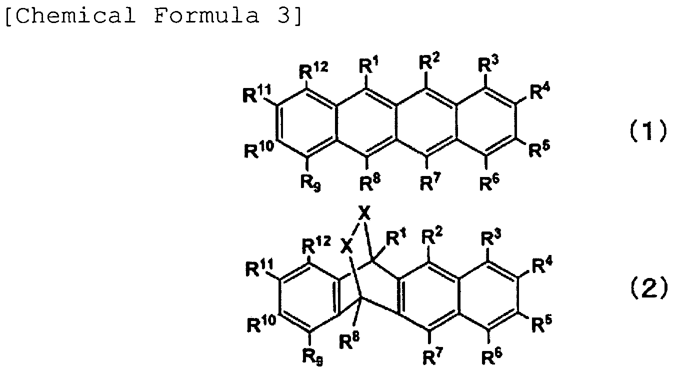 in the general formulas (1) and (2), R¹ to R¹² each may be the same or different and are selected from among hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a cyano group, a carbonyl group, a carboxyl group, an oxycarbonyl group, a carbamoyl group, an amino group, a silyl group, a phosphine oxide group and a condensed ring formed by combining adjacent substituents with each other, and in the general formula (2), X is an atom or an atomic group selected from among C=O, CH₂, O and CHR²¹, and R²¹ is a substituent selected from among an alkyl group, an alkenyl group, an alkoxy group and an acyl group and R²¹s may have a bond therebetween to form a ring.

7. The method for producing an organic EL element according to claim 6, wherein the amount of the naphthacene precursor compound present in the emissive layer and represented by the general formula (2) is 1.0 part by weight or less with respect to 100 parts by weight of the naphthacene derivative represented by the general formula (1).

8. The method for producing an organic EL element according to claim 6 or 7, wherein the amount of the naphthacene precursor compound present in the emissive layer and represented by the general formula (2) is 0.001 parts by weight or more with respect to 100 parts by weight of the naphthacene derivative represented by the general formula (1).

9. The method for producing an organic EL element according to any one of claims 6 to 8, comprising the steps of forming a layer containing at least the naphthacene precursor compound represented by the general formula (2) on a donor substrate; converting the naphthacene precursor compound represented by the general formula (2) on the donor substrate to the naphthacene derivative represented by the general formula (1) by conversion treatment; and transferring the layer on the donor substrate to a device substrate of the organic EL element to form an emissive layer.

10. The method for producing an organic EL element according to claim 9, wherein the method of forming the layer containing the naphthacene precursor compound represented by the general formula (2) on the donor substrate is based on a wet process.

11. The method for producing an organic EL element according to claim 9 or 10, wherein a conversion treatment is further performed after the transferring step.

12. The method for producing an organic EL element according to any one of claims 6 to 8, comprising the steps of forming a layer containing at least the naphthacene precursor compound represented by the general formula (2) on a substrate having at least an anode and a hole transporting layer; and converting the naphthacene precursor compound represented by the general formula (2) to the naphthacene derivative represented by the general formula (1) by conversion treatment to form an emissive layer.

13. The method for producing an organic EL element according to any one of claims 6 to 12, wherein the conversion treatment is light-irradiation and/or a heat treatment.
